# EUROPEAN PATENT APPLICATION

(11) **EP 3 533 801 A1**
(43) Date of publication of application: **04.09.2019**
(21) Application number: 17865765.6
(22) Date of filing: 27.10.2017
(51) Int. Cl.: C07G 99/00, C12N 5/0775, A61K 35/28, A61P 29/00

(54) **SYNOVIOLIN EXPRESSION INHIBITOR CONTAINING MESENCHYMAL STEM CELL OR CULTURE SUPERNATANT THEREOF**

(30) Priority: 31.10.2016 JP 2016213580
(71) Applicant: Biomimetics Sympathies Inc., Tokyo 135-0064 (JP)
(72) Inventor: URUSHIHATA Naoki, Tokyo 135-0064 (JP); TANEMURA Hideki, Tokyo 135-0064 (JP); TAKAGAKI Kentaro, Tokyo 135-0064 (JP); OKI Katsuyuki, Tokyo 135-0064 (JP); NAKAJIMA Toshihiro, Tokyo 160-8402 (JP); ARATANI Satoko, Tokyo 160-8402 (JP)
(74) Representative: Simpson, Tobias Rutger
(86) International application number: PCT/JP2017/039013
(87) International publication number: WO 2018/079753

(57) **Abstract**

[Problems to Be Solved by the Invention] A synoviolin expression inhibitor for inhibiting the expression of a synoviolin gene and a synoviolin protein is provided.

[Means to Solve the Problems] A synoviolin expression inhibitor, which inhibits an expression of a synoviolin gene or an expression of a synoviolin protein, the inhibitor comprising, as an active ingredient, a mesenchymal stem cell or a culture supernatant thereof and a method of producing a synoviolin expression inhibitor comprising the step of obtaining a mesenchymal stem cell or a culture supernatant thereof by culturing a mesenchymal stem cell using a serum-free medium, and the step of preparing the synoviolin expression inhibitor, which inhibits an expression of a synoviolin gene or an expression of a synoviolin protein, by using the mesenchymal stem cell or the culture supernatant thereof obtained in the previous step.

## Description

### [Field of the Invention]

The present invention relates to a synoviolin expression inhibitor comprising a mesenchymal stem cell or a culture supernatant thereof.

### [Background of the Invention]

WO 2002-52007 pamphlet (Non-patent document 1 mentioned below) describes synoviolin (SYVN1: Synovial Apoptosis Inhibitor 1) and a gene which codes for synoviolin.

Synoviolin is a protein which has been identified as a factor overexpressing in synovial cells from a rheumatoid patient (Non-patent document 1). Synoviolin functions as E3 ligase for ubiquitination. An inhibitor of E3 ligase activity of synoviolin has been already discovered and the use of the inhibitor suppresses symptoms of rheumatoid arthritis (Non-patent document 2). It has been also revealed that, besides rheumatism, synoviolin promotes an excessive secretion of collagen in the lung and lever or the like, causing fibrosis (Non-patent documents 3, and 4). Further, it has been suggested that synoviolin could cause carcinogenesis because synoviolin is involved in a decomposition of anti-cancer p53 gene (Non-patent document 5).

In view of the facts that synoviolin-heteroknockout mouse rarely develops rheumatism (Non-patent document 1), and that an increase in the expression of synoviolin in the peripheral blood is observed in a rheumatoid patient (Non-patent document 6), it is considered to be important not only to inhibit synoviolin activity but also to control an amount of synoviolin in order to alleviate symptoms of rheumatism. So far, a method of controlling the activity of synoviolin with a chemical compound has been known, buy it has been not yet known how to control the expression of synoviolin.

### [Prior Documents]

### [Patent Documents]

[Patent document 1] WO 2002-52007 pamphlet

### [Non-patent Documents]

[Non-patent document 1] Amano, T., et al. (2003). Genes & Development, 17(19), 2436-49.
[Non-patent document 2] Yagishita, N., et al. (2012). International Journal of Molecular Medicine, 30(6), 1281-6.
[Non-patent document 3] Nakajima, F., et al. (2015). International Journal of Molecular Medicine, 35(1), 110-6.
[Non-patent document 4] Hasegawa, D., et al. (2010). PloS One, 5(10), e13590.
[Non-patent document 5] Yamasaki, S., et al. (2007). The EMBO Journal, 26(1), 113-22.
[Non-patent document 6] Toh, M.-L., et al. (2006). Arthritis and Rheumatism, 54(7), 2109-18
[Non-patent document 7] Le Blanc, K., & Mougiakakos, D. (2012). Nature Reviews Immunology, 12(5), 383-396.

### [Summary of the Invention]

### [Problems to Be Solved by the Invention]

Thus, an object of the present invention is to provide a synoviolin expression inhibitor for inhibiting the expression of synoviolin gene and synoviolin protein.

### [Means to Solve the Problems]

The present invention is based on experimental findings that a mesenchymal stem cell or a culture supernatant thereof effectively inhibits the expression of synoviolin gene and synoviolin protein.

The first aspect of the present invention relates to a synoviolin expression inhibitor. The synoviolin expression inhibitor is a medicine to inhibit the expression of synoviolin gene and synoviolin protein. The synoviolin expression inhibitor of the present invention comprises an effective amount of the mesenchymal stem cell or the culture supernatant thereof as an active ingredient.

### [Effects of the Invention]

The present invention provides a synoviolin expression inhibitor to inhibit the expression of synoviolin gene and synoviolin protein.

### [Brief Explanation of the Drawings]

Fig.1 is a graph, which substitutes a figure, showing measurement results of the number of cells.
Fig.2 is a graph, which substitutes a figure, showing an expression of synoviolin gene.
Fig.3 is a graph, which substitutes a figure, where thicknesses measured are plotted with an initial thickness of the hind paw before collagen sensitization being set to be zero.
Fig.4 is a graph, which substitutes a figure, showing expression of the synoviolin genes in various cell lines.

### [Embodiments for Carrying Out the Invention]

The embodiments for carrying out the present invention are explained below with reference to the drawings. The present invention is not limited to the embodiments explained below and embodiments appropriately modified by a person having ordinary skills in the art based on the following embodiments are also encompassed in the invention.

The present invention is based on the experimental findings that a mesenchymal stem cell or a culture supernatant thereof effectively inhibits the expression of synoviolin gene and synoviolin protein.

The first aspect of the present invention relates to a synoviolin expression inhibitor. The synoviolin expression inhibitor is a medicine to inhibit the expression of synoviolin gene and synoviolin protein. The synoviolin expression inhibitor of the present invention comprises an effective amount of the mesenchymal stem cell or the culture supernatant thereof as an active ingredient. The culture supernatant means a supernatant obtained by culturing mesenchymal stem cells using a medium, which preferably comprises the mesenchymal stem cell.

The mesenchymal stem cells are somatic stem cells having pluripotency and are present in adipose, bone marrow, umbilical cord, amnion, placenta, dental pulp, or the like of the living body (Non-patent document 7). The mesenchymal stem cells can differentiate into various mesoderm cells, for example, osteoblasts, chondrocytes, and adipocytes. Because of its pluripotency, the mesenchymal stem cells are expected to be applied to regenerative medical treatment of bone, joint, muscle, liver, kidney, heart, central nervous system, and pancreas. In addition to supplementation and substitution of damaged tissues, the mesenchymal stem cells perform various useful functions in vivo by producing various cytokines and extracellular matrices. In particular, studies on the immunosuppressive ability of the mesenchymal stem cells have progressed, and in Canada and New Zealand, allogenic mesenchymal stem cells isolated from the bone marrow are approved as a therapeutic agent for graft versus host disease (GvHD).

The mesenchymal stem cells may be cultured by a serum-free culture method using a serum-free medium. The serum-free culture method of mesenchymal stem cells is described in, for example, Japanese Patent Application Laid-open No.2012-157263.

As explained above, inhibiting the expression and/or functions of synoviolin protein contributes to the treatment of rheumatism. The synoviolin expression inhibitor of the present invention, therefore, can be effectively used as a therapeutic agent or prophylactic agent for rheumatism. Further, the present invention can supress the expression of synoviolin, so that it can provide a functional food capable of suppressing synoviolin expression. An example of the food is anti-obesity food, such as supplement or food additive.

Japanese Patent Application Laid-open No.2009-155204 describes that suppressing the expression of a gene which codes synoviolin can regulate productions of IL-4 and IgE, and is effective for preventing and treating allergic diseases, infectious diseases, autoimmune diseases, and leukaemia. In addition, this document discloses, as an example of the synoviolin coding gene, a gene comprising the nucleotide sequence of SEQ ID No.1 shown in the document. Accordingly, the synoviolin expression inhibitor of the present invention can be used as an inhibitor of IL-4 production or IgE production and as prophylactic or therapeutic agent for allergic diseases, infectious diseases, autoimmune diseases or leukaemia.

WO2006/137514 pamphlet describes a cancer therapeutic agent comprising, as an active ingredient, a substance which inhibits the expression or functions of synoviolin. Accordingly, the synoviolin expression inhibitor of the present invention can be effectively used as a cancer therapeutic agent.

In the present invention, a medium comprises DMEM, MCDB 201, alpha-MEM, F12 or a mixture thereof as a basal medium and may comprises 1 to 20% of bovine or human serum. Alternatively, a known serum-free medium may be used. The medium in the present invention may comprise a component of a known medium. Known elements described in published documents such as Japanese Patent No.4385076 (Patent document 1), and Japanese Patent Application Laid-open No.2012-157263, both of which disclose serum-free medium for serum- free culture of animal cells.

Methods of preparing the medium are known, and those described in, for example, the aforementioned patent documents and books on cell culture may be appropriately employed.

Examples of the medium include serum-free media comprising DMEM, MCDB 201, alpha-MEM, F12 and a mixture thereof each as a basal medium.

As a method of culturing, a known method can be appropriately employed. Based on an example of culturing the mesenchymal stem cells, the method of culturing the cells is explained below. Firstly, subcutaneous adipose tissues are obtained by surgically excising a subject or sucking from a subject. From the subcutaneous adipose tissues thus obtained, a stromal vascular fraction (SVF) is then extracted. From the stromal vascular fraction, mesenchymal stem cells are obtained by using a solution of enzymes for tissue dissociation. Amounts of the stromal vascular fraction (SVF) and the solution of enzymes for tissue dissociation may be adjusted with reference to the examples explained later in the specification. Reaction temperature may be appropriately adjusted in the range of from 25 °C to 40 °C, and reaction time may be appropriately adjusted in the range of from 15 mins to 5 hours. After having visually detected the dissociation of the tissues, a cell pellet is recovered by, for example, centrifugation. The pellet thus recovered is suspended in physiological saline or the like, and the suspension thus obtained is subjected to filtration. The filtrate is centrifuged again to obtain the mesenchymal stem cells as a secondary pellet.

Culturing is performed using the pellets obtained as described above. A medium for a primary culture and those for subsequent cultures may be the same or different. For example, an adhesion culture is performed using the medium as mentioned above in a container such as a flask or a petri dish. For example, the subculture is repeated at a confluence of from 90 to 95 % and expressions of stem cell markers such as CD73, CD90, or CD105 and CD10 are determined even during the subculture as necessary by slow cytometer or immunostaining method. The subculture may be performed by recovering a culture supernatant, and then exfoliating cells with an exfoliating agent followed by recovering cell pellets by centrifugation and seeding a suspension of the pellets in a medium in a flask. By this process, the culture supernatant can be obtained. In about 3 weeks from the start of the primary culture, a cell population comprising 1 x 10⁸ order of the adipose-derived mesenchymal stem cells can be obtained. Subsequently, the cell preparation thus obtained may be tested for quality control standard items as necessary such as endotoxin concentration, sterility test, specific virus infection negative test, mycoplasma negative test, viability, and so on.

The agent of the present invention may be prepared by a method in the art. The agent of the present invention may be formulated as an oral agent or a parenteral agent, preferably a parenteral agent. Such parenteral agent may be formulated in a liquid agent (such as aqueous liquid agent, non-aqueous liquid agent, suspension agent, and emulsion agent) or a solid agent (such as solidified powder agent, and freeze-dried agent). The agent of the present invention may be formulated as a sustained release agent.

The liquid agent may be prepared by a known method. For example, it can be prepared by dissolving mesenchymal stem cells in a pharmaceutically acceptable solvent and then poring the solution in a container for liquid agent. Examples of the pharmaceutically acceptable solvent include water for injection, distilled water, physiological saline solution, and electrolyte solution agent. Preferably, a sterilized container is used such as a sterilized ampoule, a vial and a bag. The container may be a known container made of glass or plastic. Examples of the plastic container include those made of polyvinyl chloride, polyethylene, polypropylene, or ethylene/vinyl acetate copolymer. Examples of method of sterilizing the container and solvent include heating method (flame method, drying method, high-temperature vapor method, circulation vapor method, boiling method, or the like), filtration method, irradiation method (radiation method, ultraviolet method, high frequency method, or the like), gas method, and chemical sterilization method. A person with ordinary skills in the art can select an appropriate method depending on the material of the container and properties of the solvent.

The solid agent may be prepared by freeze drying method, spray drying method or sterile recrystallization method.

The present invention can be provided as a kit of a combination of the agent comprising the mesenchymal stem cells or the culture supernatant thereof of the present invention and a medical device. Examples of the kid include a medical device such as a syringe filled with the agent comprising the mesenchymal stem cells of the present invention, and a soft bag having a compartment filled with the solid agent and another compartment filled with the solvent with a partition therebetween being capable of providing an opening to allow the solid agent and the solvent to be mixed in use. These kits are preferably used because they not only facilitate healthcare professionals to prepare the agent but also prevent contamination with bacteria or foreign materials. These syringe and soft bag are known, and healthcare professionals can appropriately use them.

The agent comprising the mesenchymal stem cells or the culture supernatant thereof of the present invention can be administered by a known method such as intravenous administration, intraarterial administration, intramuscular administration, subcutaneous administration, intraperitoneal administration or intra-nasal administration. A preferred administration method is administration by injection, and the agent of the present invention can be administered also by intravenous drip infusion. The agent of the present invention may be directly injected into an affected part or a target site, or it can be administered by surgically incising an affected part.

The agent of the present invention may be prepared from the active ingredient of the mesenchymal stem cells or the culture supernatant thereof in combination with a pharmaceutically acceptable carrier or medium. Examples of the pharmaceutically acceptable carrier or medium include pharmaceutically acceptable substances such as excipients, stabilizers, solubilizing agents, emulsifiers, suspending agents, buffer agents, isotonic agents, antioxidants, and preservatives. Polymer material such as polyethylene glycol (PEG) and a clathrate compound such as cyclodextrin or the like can be used. Examples of the excipients include substances that are pharmacologically inactive such as starch and lactose. Examples of the stabilizers include albumin, gelatin, sorbitol, and mannitol, lactose, sucrose, trehalose, maltose, and glucose, among which sucrose and trehalose are preferred. Examples of the solubilizing agents include ethanol, glycerin, propylene glycol, and polyethylene glycol. Examples of the emulsifiers include lecithin, aluminum stearate, and sorbitan sesquioleate. Examples of the suspending agents include polyethylene glycol (available under the trade name of "Macrogol"), polyvinylpyrrolidone (PVP), and carmellose (CMC). Examples of the isotonic agents include sodium chloride and glucose. Examples of buffer agents include citrate, acetate, boric acid, and phosphate. Examples of antioxidants include ascorbic acid, sodium hydrogen sulfite, and sodium pyrosulfite. Examples of preservatives include phenol, thimerosal, and benzalkonium chloride.

The mesenchymal stem cells, which is a main component of the agent of the present invention, may be contained in the agent of the present invention in an effective amount, which varies depending on subject type, age, symptoms or the like. In general, an example of a daily dose, represented in the number of mesenchymal stem cells per kg of body weight, rages from 1 x 10⁴ to 5 x 10⁷ cells/kg and may range from 1 x 10⁵ to 5 x 10⁶ cells/kg. Preferably, the daily dose is divided in 2 to 5 parts. Alternatively, the agent of the present invention is formulated as a sustained release agent to reduce the number of administrations. The sustained release agent may be prepared by a known method. By administering a divided dosage or administrating in the form of sustained release agent, an in-vivo concentration of the agent can be maintained more easily, so that the efficacy can be maintained more easily. In addition, a side effect can decrease to reduce a burden on the patient.

The present invention also provides the use of the aforementioned mesenchymal stem cells of the present invention for preparing a prophylactic or a therapeutic agent for various diseases. That is, as explained above, the prophylactic or the therapeutic agent for various diseases as described above can be prepared by obtaining the mesenchymal stem cells and using thus obtained mesenchymal stem cells.

The present invention also provides a method of treating and a method of preventing various diseases. The method of treating various diseases comprises the step of administrating to patients affected by the various diseases an effective amount of the agent comprising, as the active ingredient, the mesenchymal stem cells of the present invention prepared as described above. The method of preventing various diseases comprises the step of administrating to subjects liable to contract various diseases an effective amount of the agent comprising, as the active ingredient, the mesenchymal stem cells of the present invention prepared as described above.

The culture supernatant of the present invention may comprise mesenchymal stem cells. When the mesenchymal stem cells are contained, they may be present in an amount of 1/100 to 1/1 of the content in the aforesaid agent comprising the mesenchymal stem cells. Examples of the culture supernatant include a supernatant obtained by centrifuging a culture supernatant (liquid?) to sperate liquid from solid, a dehydrated supernatant treated by freeze drying to remove water, a concentrated supernatant treated by an evaporator or the like under reduced pressure, a concentrated supernatant treated by a ultrafiltration membrane or the like, and a supernatant treated by a filter to separate liquid and solid. For example, the culture super natant may be prepared by the following method: a culture supernatant(liquid?) is centrifuged (for example, at 3,000 rpm for 10 minutes); the supernatant thus obtained is filtered by using a syringe filter (for example, 0.45 µm); the filtrate is subjected to fractionation with ammonium sulfate (for example, 65% saturated ammonium sulfate), and the precipitate is subjected to dialysis using a saline solution. The culture supernatant obtained may be used as it is or freeze-dried, stored and thawed in use. A pharmacologically acceptable carrier may be added to the culture supernatant in such an amount to increase a volume of the supernatant to an amount, for example, 0.2 ml or 0.5 ml for easy handling.

A preferred embodiment of this aspect is an agent comprising the culture supernatant as an active ingredient. More specific preferred embodiment of this aspect is an agent comprising the culture supernatant as an active ingredient, wherein the agent is a therapeutic agent or a prophylactic agent for the aforesaid diseases. An agent comprising a culture supernatant as an active ingredient is known, for example, from Japanese Patent No.5139294, and Japanese Patent No.5526320. Accordingly, the agent of the present invention comprising the culture supernatant can be prepared by a known method.

The present invention also provides the use of the aforesaid culture supernatant of the present invention for preparing a therapeutic agent or a prophylactic agent for the aforesaid diseases. That is, the prophylactic or the therapeutic agent for various diseases as described above can be prepared by obtaining the culture supernatant of the mesenchymal stem cell and using thus obtained culture supernatant of mesenchymal stem cell.

The present invention also provides a method of treating various diseases and a method of preventing various diseases. The method of treating various diseases comprises the step of administrating to patients affected by the various diseases an effective amount of the agent comprising, as the active ingredient, the culture supernatant of the present invention prepared as described above. The method of preventing various diseases comprises the step of administrating to subjects liable to contract various diseases an effective amount of the agent comprising, as the active ingredient, the culture supernatant of the present invention prepared as described above.

### [Example 1]

### Study of influences of a supernatant of mesenchymal stem cells on a proliferation of synovial cells

Firstly, a culture supernatant was obtained by culturing mesenchymal stem cells derived from adipose for 3 days in a serum-free medium for mesenchymal stem cells (sf-DOT medium, trade name?) available from BioMimetics Sympathies Inc.

Synovial cells collected from a rheumatoid patient were seeded on a 96-well cell culture plate at a density of 3x10³ cells/well. On the next day, the culture medium was replaced with the culture supernatant of the mesenchymal stem cells. As a negative control, a culture was performed in the sf-DOT medium that had not been used for culturing mesenchymal stem cells. On the 3rd day from the replacement of the medium, the number of the cells was counted using Cell Counting Kit-8 (Dojindo Molecular Technologies, Inc.).

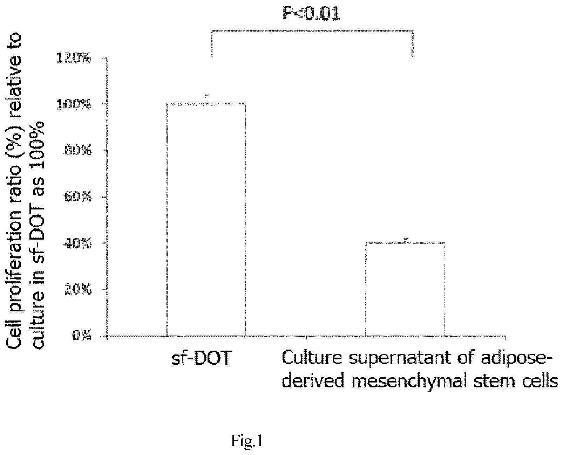

The results are shown in Fig.1, which is the graph showing the measurement results of the number of the cells. From Fig. 1, it was shown that the culture supernatant of the adipose-derived mesenchymal stem cells suppresses the proliferation of the synovial cells from the rheumatoid patient.

### [Example 2]

### Study of influences of a supernatant of mesenchymal stem cells on synoviolin expression in synovial cells

In this example, an activity of suppressing synoviolin expression in synovial cells from a rheumatoid patient was measured using a culture supernatant of mesenchymal stem cells derived from adipose, umbilical cord, or amnion. Firstly, a culture supernatant was obtained by culturing adipose-derived mesenchymal stem cells in the sf-DOT medium for 3 days. Synovial cells collected from the rheumatoid patient were seeded on a 6-well cell culture plate at a density of 1x10⁵ cells/well.

On the next day, the medium was replaced with the supernatant of mesenchymal stem cells. As a negative control, a culture was performed in the sf-DOT medium that had not been used for culturing mesenchymal stem cells. On the 3rd day of the culture, the cells were recovered, and RNA was extracted. From the RNA, cDNA was synthesized and synoviolin expression was measured by quantitative PCR. As an internal standard, RPLP0 gene expression was quantified to calibrate the expression of the synoviolin gene.

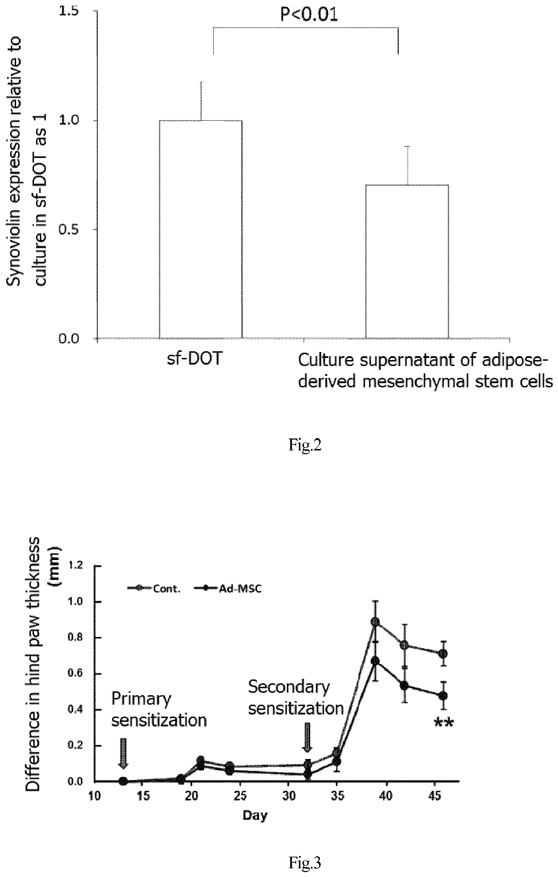

The results are shown in Fig.2, which is the graph showing the expression of synoviolin gene. As shown in Fig.2, the culture supernatant of the mesenchymal stem cells derived from adipose etc. suppressed the synoviolin gene expression in the synovial cells from the rheumatoid patient.

### [Example 3]

### Study of influences of the mesenchymal stem cells on an arthritis model

Influences of the mesenchymal stem cells derived from human adipose on a collagen-induced arthritis model using DBA/1 mice (body weight of about 25 g) were studied. The DBA/1 mice were sensitized by injecting intradermally at the tail base bovine type II collagen mixed with an adjuvant. Two weeks after the primary collagen sensitization, the secondary collagen sensitization was performed to induce arthritis. From two weeks before the primary sensitization, 1x10⁶ cells of the mesenchymal stem cells were intradermally administered at the back of the mice two times per week for 9 weeks. During the observation period, a thickness of the hind paw was measured with a vernier caliper.

The mesenchymal stem cells used were those cultured in Example 1.

Fig.3 shows a graph in which thicknesses measured are plotted with a thickness of the hind paw before the collagen sensitization being set to be zero. As can be seen from Fig.3, collagen-induced arthritis was suppressed in the group that received the mesenchymal stem cells.

### [Example 4]

### Study of influences of the culture supernatant of mesenchymal stem cells on synoviolin expression in various cell lines

In this example, an activity of suppressing synoviolin expression in cell lines generally used for researches was measured using a culture supernatant of mesenchymal stem cells derived from adipose, umbilical cord, or amnion. Firstly, adipose-derived mesenchymal stem cells were cultured in the sf-DOT medium for 3 days and a culture supernatant was obtained. On a 6-well culture plate, A549, HEK293 and HeLa cells were respectively seeded at a density of 1x10⁵ cells/well.

On the next day, the culture medium was replaced with the culture supernatant of the mesenchymal stem cells. As a negative control, a culture was performed in the sf-DOT medium that had not been used for culturing stem cells. On the 3rd day of the culture, the cells were recovered, and RNA was extracted. From the RNA, cDNA was synthesized and synoviolin gene expression was measured by quantitative PCR. As an internal standard, RPLP0 gene expression was quantified to calibrate the expression of the synoviolin gene.

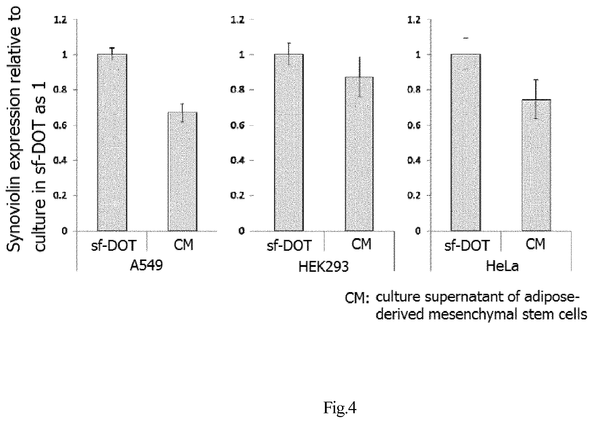

The results are shown in Fig.4, which is the graph showing the expression of synoviolin gene. As shown in Fig.4, the culture supernatant of the mesenchymal stem cells derived from adipose suppressed the synoviolin gene expression in A549, HEK293 and HeLa cells.

### [Industrial Applicability]

Synoviolin is being studied for various pharmaceutical applications, and the present invention can be used in the pharmaceutical industry. Especially, the present invention can inhibit the expression of synoviolin. Accordingly, treatment agents for various diseases involving synoviolin and various types of food can be provided.

## Claims

1. A synoviolin expression inhibitor, which inhibits an expression of a synoviolin gene or an expression of a synoviolin protein, the inhibitor comprising, as an active ingredient, a mesenchymal stem cell or a culture supernatant thereof.

2. A method of producing a synoviolin expression inhibitor comprising the steps of:
obtaining a mesenchymal stem cell or a culture supernatant thereof by culturing a mesenchymal stem cell; and
preparing the synoviolin expression inhibitor, which inhibits an expression of a synoviolin gene or an expression of a synoviolin protein, by using the mesenchymal stem cell or the culture supernatant thereof obtained in the previous step.
